# DEMANDE DE BREVET EUROPEEN

(11) **EP 4 620 456 A2**
(43) Date de publication de la demande: **24.09.2025**
(21) Numéro de dépôt: 25178004.5
(22) Date de dépôt: 31.07.2020
(51) Int. Cl.: A61K 9/08

(54) **COMPOSITIONS VÉTÉRINAIRES POUR PRÉVENIR ET/OU TRAITER LA CRYPTOSPORIDIOSE**

(30) Priorité: 01.08.2019 EP 19305996
(62) Demande divisionnaire de: 20746228.4
(71) Demandeur: Ceva Santé Animale, 33500 Libourne (FR)
(72) Inventeur: BESCHE, Béatrice, 33500 LIBOURNE (FR); JIMENEZ, Catherine, 33500 LIBOURNE (FR)
(74) Mandataire: Cabinet Becker et Associés

(57) **Abrégé**

La présente invention porte sur une composition vétérinaire comprenant de la paromomycine ou un de ses sels pharmaceutiquement acceptables destinée à être utilisée dans la prévention et/ou le traitement de la cryptosporidiose chez un mammifère non-humain, dans laquelle la composition est administrée audit mammifère non-humain à une dose de paromomycine comprise entre 80 et 140 mg/kg/jour pendant 3 à 6 jours.

## Description

### OBJET DE L'INVENTION

La présente invention concerne le domaine vétérinaire et, plus particulièrement, l'utilisation de compositions antibiotiques pour traiter la cryptosporidiose chez les mammifères non-humains, de préférence chez les jeunes bovins.

### ARRIERE-PLAN TECHNOLOGIQUE DE L'INVENTION

La cryptosporidiose est une affection zoonotique, cosmopolite due à un protozoaire du genre *Cryptosporidium,* dont l'importance médicale et économique est majeure. En effet, *Cryptosporidium* est un parasite à très large distribution, avec de grandes capacités de multiplication et de dissémination. Il contamine les mammifères, les reptiles, les oiseaux, et les poissons essentiellement via un contact direct avec de l'eau, des animaux ou des aliments infectés. *Cryptosporidium parvum* est l'espèce zoonotique la plus répandue et est responsable d'infections chez la plupart des mammifères dont les ruminants, ainsi que de la majorité des contaminations par l'eau. Les ruminants sont la principale source de contamination pour l'Homme.

La cryptosporidiose présente donc un enjeu majeur de santé publique mondiale. Estimée responsable de 30-50% des décès des enfants de moins de cinq ans, la cryptosporidiose est considérée comme la seconde cause majeure de diarrhée et de mort chez les enfants, après le rotavirus. Des études rapportent des séquelles du développement cognitif et physique chez des enfants de cinq ans après avoir eu la cryptosporidiose.

Chez les animaux de rente, le parasite intestinal *Cryptosporidium* et la maladie qui en est associée, à savoir la cryptosporidiose, sont responsables de sévères troubles digestifs et sont à l'origine de pertes économiques importantes (morbidité élevée, retards de croissance, frais vétérinaires, temps de travail supplémentaire). Il est estimé en Europe que la cryptosporidiose touche 20 à 40 % des jeunes veaux dans les 2-3 premières semaines de vie.

L'expression clinique de la cryptosporidiose est non spécifique, mais se développe avant 21 jours d'âge. Elle se caractérise par une diarrhée aiguë plus ou moins intense, une déshydratation, une léthargie, une anorexie et une douleur abdominale. Une perte de poids, un retard de croissance et de la fièvre sont également observés. L'excrétion des oocystes débute vers le 4ème jour de vie, puis présente un pic entre le 7^{ème} et le 18^{ème} jour avant de diminuer ensuite. Plus la dose infectante d'oocystes est importante et précoce, plus l'excrétion des oocystes et la diarrhée seront importantes et longues. Une corrélation est démontrée entre, d'une part l'excrétion d'oocystes et la diarrhée, et d'autre part entre le taux de mortalité à 90 jours et une diarrhée sévère associée à une forte excrétion. La transmission et l'extension rapide de la maladie sont assurées par les oocystes, très résistants dans leur environnement.

Devant ce fléau, divers traitements impliquant de nombreuses molécules ont été testées pour leur efficacité contre la cryptosporidiose. Par exemple, plusieurs études ont démontré l'efficacité du lasalocide contre la cryptosporidiose chez le veau. Cependant, la dose minimale efficace (3 mg/kg/jour) est très proche de la dose toxique (5 mg/kg/jour). Son indice thérapeutique est très médiocre et son utilisation est à l'origine d'effets secondaires fréquents et sévères (anorexie, tachycardie, tachypnée, anorexie, paralysie et mort). Cette toxicité et cette dangerosité, plus marquées encore chez les veaux de moins de 7 jours, réduisent donc l'utilisation pratique de cette molécule. Le nitazoxanide, molécule de la famille des thiazolides, a aussi a témoigné de son efficacité contre les cryptosporidies du veau. En revanche, il a aussi été observé une persistance de la diarrhée fréquente, et de nombreux effets secondaires et parfois même une forte mortalité associée aux traitements à base de nitazoxanide. Il est supposé que ces effets secondaires seraient dus à l'action de la molécule sur la flore bactérienne commensale. Au vu de ces effets, l'utilisation de cette molécule n'est pas recommandée chez les animaux de rente.

Aydogdu et al. se sont également intéressé à deux autres molécules, le lactate d'halofuginone, dérivé de la famille des quinazolinones, et la paromomycine, en comparant leur efficacité dans le traitement de veaux naturellement infectés avec *Cryptosporidium parvum.* Plus particulièrement, Aydogdu et al. ont montré une efficacité thérapeutique d'une dose de lactate d'halofuginone à 100 µg/kg/jour pendant 7 jours et d'une dose de sulfate de paromomycine à 100 mg/kg/jour, correspondant à une dose de paromomycine à 70 mg/kg/jour (Gabbrocol^{®}), pendant 7 jours. Toutefois, ces molécules aux doses testées ne sont pas en mesure d'éliminer complètement l'infection et seraient donc plutôt adaptées à des visées prophylactiques. En outre, ces traitements continus d'une période longue d'au minimum 7 jours sont relativement contraignants dans leur application pour le vétérinaire ou l'éleveur, et peuvent aussi laisser apparaitre des problèmes de résistance.

Malgré certains résultats prometteurs, il n'existe à ce jour aucun traitement capable de contrôler durablement les signes cliniques et l'infection parasitaire. L'utilisation de certaines drogues peut permettre la diminution de l'excrétion des oocystes et des signes cliniques sans pour autant éradiquer totalement le parasite. En outre, l'infection parasitaire est très souvent corrélée d'une perte de poids significative dommageable sur l'impact nutritionnel et la croissance des animaux de rente.

Ainsi, il persiste aujourd'hui un besoin de développer de nouveaux traitement efficaces et non résistants permettant à la fois de traiter la cryptosporidiose et d'améliorer les signes cliniques liés à cette maladie parasitaire.

### RESUME DE L'INVENTION

Dans ce contexte, les inventeurs ont proposé un nouveau traitement plus efficace pour prévenir et/ou traiter la cryptosporidiose chez un mammifère non-humain. Plus précisément, les inventeurs ont démontré qu'une utilisation de sulfate de paromomycine à des doses plus élevées, c'est-à-dire supérieures à 100 mg/kg/jour (correspondant à des doses supérieures à 70 mg/kg/jour de paromomycine), permettaient d'éradiquer efficacement et plus rapidement l'infection parasitaire, et par la même occasion d'améliorer rapidement les signes cliniques du mammifère non-humain souffrant de cryptosporidiose. Ces traitements efficaces de durée plus courte, en plus d'être moins contraignants pour l'utilisateur, ont pour avantage de diminuer les risques de contamination et de propagation entre mammifères non-humains, en particulier issus d'un même élevage. Ces traitements efficaces de durée plus courte peuvent aussi limiter l'apparition de parasites résistants à la paromomycine.

La présente invention concerne donc une composition vétérinaire comprenant de la paromomycine ou un de ses sels pharmaceutiquement acceptables destinée à être utilisée dans la prévention et/ou le traitement de la cryptosporidiose chez un mammifère non-humain, dans laquelle la composition est administrée audit mammifère non-humain à une dose de paromomycine comprise entre 80 et 140 mg/kg/jour pendant 3 à 6 jours.

Selon un mode particulier de l'invention, la dose de paromomycine est comprise entre 84 et 126 mg/kg/jour, entre 91 et 119 mg/kg/jour, de préférence entre 98 et 112 mg/kg/jour. Selon un mode préféré de l'invention, la dose de paromomycine est d'environ 105 mg/kg/jour.

Selon un autre mode particulier de l'invention, le sel pharmaceutiquement acceptable de la paromomycine est un sulfate.

Un autre objet de l'invention concerne donc une composition vétérinaire comprenant du sulfate de paromomycine destinée à être utilisée dans la prévention et/ou le traitement de la cryptosporidiose chez un mammifère non-humain, dans laquelle la composition est administrée audit mammifère non-humain à une dose de sulfate de paromomycine comprise entre 114,3 et 200 mg/kg/jour pendant 3 à 6 jours. Selon un mode préféré, la dose de sulfate de paromomycine est d'environ 150 mg/kg/jour.

Selon un autre mode particulier, la composition utilisée selon la présente invention est administrée pendant 3 à 5 jours, de préférence pendant 5 jours. De préférence, la composition est administrée en une seule prise par jour.

Selon un autre mode particulier, la composition utilisée selon la présente invention est administrée par voie orale.

Selon un mode préféré de l'invention, le mammifère non-humain est un bovin, un porcin, un caprin, ou un ovin, de préférence un bovin. Selon un mode encore plus préféré, le mammifère non-humain est un nouveau-né ou ayant un âge allant jusqu'à 21 jours, de préférence jusqu'à 14 jours, de manière encore plus préférée ayant un âge de 2 ou 3 jours ou un âge compris entre 6 et 13 jours, de préférence entre 7 et 10 jours. Avantageusement, le mammifère non-humain est un veau.

Selon un autre mode particulier, la composition utilisée selon la présente invention comprend en outre au moins un excipient. Selon un autre mode particulier de l'invention, la composition est une solution.

Un autre objet de l'invention concerne l'utilisation d'une composition telle que définie dans la présente demande pour réduire et/ou supprimer l'excrétion des oocystes. Un autre objet de l'invention concerne l'utilisation d'une composition telle que définie dans la présente demande pour réduire et/ou supprimer l'état de déshydratation. Un objet supplémentaire de l'invention concerne l'utilisation d'une composition telle que définie dans la présente demande pour traiter et/ou prévenir la diarrhée. Un autre objet supplémentaire de l'invention concerne l'utilisation d'une composition telle que définie dans la présente demande pour augmenter le gain en poids journalier.

### LEGENDE DES FIGURES

**Figure 1** : Evolution du score fécal de Dt0 à Dt5 pour les groupes contrôle, 75, 100, et 150.
**Figure 2** : Evolution du score d'hydratation de Dt0 à Dt5 pour les groupes contrôle, 75, 100, et 150.
**Figure 3** : Evolution du nombre d'oocystes moyen de Dt0 à Dt10 pour les groupes contrôle, 75, 100, et 150.

### DESCRIPTION DETAILLEE DE L'INVENTION

L'invention telle que décrite dans la présente demande concerne l'utilisation journalière, d'une composition vétérinaire comprenant de la paromomycine ou un de sels pharmaceutiquement acceptables à des doses plus élevées en paromomycine que celles couramment utilisées dans l'art antérieur, c'est-à-dire supérieures à 70 mg/kg/jour, de préférence entre 80 et 140 mg/kg/jour pour prévenir et/ou traiter la cryptosporidiose chez un mammifère non-humain. L'invention concerne aussi une composition vétérinaire telle que décrite dans la présente demande pour son utilisation pour éradiquer ou éliminer *Cryptosporidium parvum* chez un mammifère non-humain, de préférence souffrant de cryptosporidiose. L'invention concerne aussi une méthode pour éradiquer ou éliminer *Cryptosporidium parvum,* comprenant l'administration d'une composition vétérinaire telle que décrite dans la présente demande chez un mammifère non-humain, de préférence souffrant de cryptosporidiose.

La présente invention porte donc sur une composition vétérinaire comprenant de la paromomycine ou un de ses sels pharmaceutiquement acceptables destinée à être utilisée dans la prévention et/ou le traitement de la cryptosporidiose chez un mammifère non-humain, dans laquelle la composition est administrée audit mammifère non-humain à une dose de paromomycine comprise entre 80 et 140 mg/kg/jour pendant 3 à 6 jours. La présente invention concerne également la paromomycine ou un de ses sels pharmaceutiquement acceptables destinée à être utilisée dans la prévention et/ou le traitement de la cryptosporidiose chez un mammifère non-humain, dans laquelle la paromomycine ou un de ses sels pharmaceutiquement acceptables est administrée audit mammifère non-humain à une dose de paromomycine comprise entre 80 et 140 mg/kg/jour pendant 3 à 6 jours. L'invention porte aussi sur une méthode pour traiter la cryptosporidiose chez un mammifère non-humain, comprenant l'administration d'une dose de paromomycine ou un de ses sels pharmaceutiquement acceptables comprise entre 80 et 140 mg/kg/jour de paromomycine ou d'une composition vétérinaire comprenant ladite dose, pendant 3 à 6 jours chez ce mammifère non-humain. L'invention porte également sur une utilisation d'une dose de paromomycine ou un de ses sels pharmaceutiquement acceptables comprise entre 80 et 140 mg/kg/jour de paromomycine pour la fabrication d'une composition vétérinaire pour traiter la cryptosporidiose chez un mammifère non-humain dans laquelle la composition est administrée audit mammifère non-humain pendant 3 à 6 jours.

Selon un mode particulier de l'invention, la dose de paromomycine est comprise entre 84 et 126 mg/kg/jour, entre 91 et 119 mg/kg/jour, de préférence entre 98 et 112 mg/kg/jour. Selon un mode préféré, la dose de paromomycine est d'environ 105 mg/kg/jour.

Dans le contexte de la présente invention, les termes « traitement » et « traiter » désignent au sens large une amélioration, une prophylaxie, une guérison d'une maladie ou d'un trouble ou signe clinique associé à la maladie, en l'occurrence la cryptosporidiose, tels que la diarrhée, la déshydratation, l'état général sur la santé, le gain en poids journalier, et le comportement. Par « traitement de cryptosporidiose », on entend également le contrôle, c'est-à-dire l'éradication, l'élimination, ou la réduction du ou des parasites responsable(s) de la cryptosporidiose, tels que *Cryptosporidium parvum,* chez un mammifère non-humain. On entend aussi le contrôle de l'excrétion des oocystes, c'est-à-dire la réduction voire la suppression de l'excrétion des oocystes du ou des parasites responsable(s) de la cryptosporidiose, tels que *Cryptosporidium parvum,* chez un mammifère non-humain.

Selon un mode particulier, ces expressions incluent le traitement curatif du mammifère non-humain contre la cryptosporidiose. Par « traitement curatif », on entend un traitement permettant la guérison du mammifère non-humain contre la cryptosporidiose. De manière particulière, le mammifère non-humain traité présente un score fécal et/ou un score sur l'état général sur la santé et/ou un score d'hydratation, et/ou un score de guérison clinique minimale, de préférence égal à 0.

Selon un autre mode particulier, ces expressions incluent le traitement préventif du mammifère non-humain contre la cryptosporidiose. Selon un premier aspect, un traitement préventif désigne un traitement réalisé avant que le mammifère non-humain ait été exposé ou ait été en contact avec l'agent causant ou à l'origine de la cryptosporidiose. Un traitement préventif réduit donc les risques pour le mammifère non-humain de développer la cryptosporidiose. Les termes « traitement » et/ou « prévention » peuvent ainsi aussi désigner la protection d'un mammifère non-humain contre la cryptosporidiose ou d'au moins un de ses troubles. Selon un second aspect, un traitement préventif désigne aussi un traitement réalisé chez un mammifère non-humain souffrant de cryptosporidiose. Le traitement réalisé chez un sujet malade peut permettre de contrôler le ou les parasite(s) à l'origine de la cryptosporidiose dans son environnement et de réduire les risques d'infection et de contamination chez les sujets sains environnants, contribuant ainsi à limiter l'expansion de la cryptosporidiose.

La paromomycine est un composé antibiotique appartenant au groupe des aminoglycosides. Elle agit sur la traduction des ARNs messagers en interrompant ainsi la synthèse protéique. Son activité antibactérienne est principalement attribuée à son interaction irréversible avec les ribosomes. La paromomycine présente un large spectre d'activité contre les bactéries Gram-positif et Gram-négatifs. Elle est ainsi notamment utilisée dans des traitements contre les infections gastro-intestinales causées par *E. coli* et *Salmonella* chez des cochons et des pré-ruminants à des doses de 25-50 mg/kg/jour pendant 3-5 jours sous la forme de son sel sulfate de paromomycine (Gabbrovet^{®}, Parofor^{®}). Comme indiqué dans l'introduction, elle est aussi utilisée contre la cryptosporidiose chez le veau mais dans des traitements utilisant des doses plus faibles que celles utilisées dans la présente invention et d'une durée plus longue, allant jusqu'à 7 jours.

Par « sels pharmaceutiquement acceptables », on entend à la fois les sels organiques et inorganiques. Des exemples représentatifs de sels inorganiques comprennent les chlorhydrates, les bromhydrates, les iodates, les sulfonates, les sulfates, et les phosphates. Des exemples représentatifs de sels organiques comprennent les formates, les acétates, les trichloroacétates, les propionates, les benzoates, les cinnamates, les fumarates, les maléates, et les méthanesulfonates. De préférence, le sel pharmaceutiquement acceptable est un sulfate. Selon un mode préféré, la paromomycine utilisée selon la présente invention est sous la forme de sulfate de paromomycine.

Selon l'invention, la paromomycine est administrée à un mammifère non-humain à une dose comprise entre 80 et 140 mg/kg/jour. Telles que décrites dans la présente demande, les doses de paromomycine correspondent à une quantité en poids de paromomycine administrée par kilogramme de poids corporel de mammifère non-humain par jour. La quantité en poids de paromomycine correspond à la quantité en poids de paromomycine en tant que telle, c'est-à-dire sans tenir compte de la forme paromomycine (comme sous forme de sel) par laquelle elle est administrée. Il est bien entendu que l'homme du métier saura adapter les quantités en fonction de la forme de paromomycine utilisée. Par exemple, une dose comprise entre 80 et 140 mg/kg/jour de paromomycine correspond à une dose de sulfate de paromomycine comprise entre 114,3 mg/kg/jour et 200 mg/kg/jour. Des doses de 80, 84, 91, 98, 105, 112, 119, 126, et 140 mg/kg/jour de paromomycine correspondent respectivement à des doses de sulfate de paromomycine de 114,3, 120, 130, 140, 150, 160, 170, 180, et 200 mg/kg/jour.

La présente invention porte donc aussi sur une composition vétérinaire comprenant du sulfate de paromomycine destinée à être utilisée dans la prévention et/ou le traitement de la cryptosporidiose chez un mammifère non-humain, dans laquelle la composition est administrée audit mammifère non-humain à une dose de sulfate de paromomycine comprise entre 114,3 et 200 mg/kg/jour, de préférence entre 120 et 200 mg/kg/jour, entre 120 et 180 mg/kg/jour, entre 130 et 170 mg/kg/jour, entre 140 et 160 mg/kg/jour, pendant 3 à 6 jours. De manière préférée, la dose de sulfate de paromomycine est d'environ 150 mg/kg/jour.

Le terme environ sera compris par l'homme du métier et peut varier dans une certaine mesure selon le contexte dans lequel il est utilisé. Si certaines utilisations de ce terme ne sont pas claires pour l'homme du métier en fonction du contexte, « environ » signifie plus ou moins 20 %, de préférence plus ou moins 10 % du terme particulier.

La paromomycine ou un de ses sels pharmaceutiquement acceptables ou les compositions les comprenant peuvent être administrés à un mammifère non-humain aux doses spécifiées dans la présente demande pendant 1 à 6 jours, 1 à 5 jours, pendant 2 à 6 jours, pendant 2 à 5 jours, pendant 3 à 6 jours, et pendant 3 à 5 jours. Dans le contexte de la présente invention, une administration pendant X à Y jours telle que définie ci-dessus signifie une administration pendant un minimum de X jours et un maximum de Y jours. En d'autres termes, le traitement est appliqué pendant au moins X jours et au plus Y jours. Une administration pendant 1 à 6 jours signifie donc une administration pendant au moins 1 jour et au plus 6 jours, c'est à dire 1, 2, 3, 4, 5 ou 6 jours. Le traitement est ensuite stoppé après l'administration effectuée au 6^{eme} jour.

Plus particulièrement, la paromomycine ou un de ses sels pharmaceutiquement acceptables ou les compositions les comprenant sont administrés à un mammifère non-humain aux doses spécifiées dans la présente demande pendant 3 à 6 jours. Ainsi, la paromomycine ou un de ses sels pharmaceutiquement acceptables sont administrés chez le mammifère non-humain à un temps T0 (Dt0) puis sont de nouveau administrés à un temps T1 (Dt1) un jour ou 24 heures après. Le traitement est ainsi répété tous les jours à partir de T0 et ce pendant 3 à 6 jours, c'est-à-dire pendant 3, 4, 5, ou 6 jours, de préférence pendant 3 à 5 jours. Selon un mode préféré de l'invention, la paromomycine ou un de ses sels pharmaceutiquement acceptables ou la composition les comprenant sont administrés pendant 3 jours, pendant 4 jours, et de préférence pendant 5 jours.

La paromomycine ou ses sels pharmaceutiquement acceptables peuvent être administrées en une ou plusieurs fois par jour, de préférence en une seule prise par jour. Selon un mode préféré de l'invention, la paromomycine ou un de ses sels pharmaceutiquement acceptables ou la composition les comprenant sont administrés en une seule prise par jour pendant 3 à 6 jours, pendant 3 à 5 jours, pendant 3 jours, pendant 4 jours, et de préférence pendant 5 jours.

Les compositions vétérinaires utilisées dans la présente invention peuvent être administrées par toutes les routes ou voies d'administration connues de l'homme du métier, telles que la voie orale ou la voie parentérale, incluant une injection par intraveineuse, intramusculaire, et sous-cutanée. Selon un mode préféré de l'invention, la composition est administrée par voie orale. Dans le cadre d'une administration orale, la composition peut être administrée directement au mammifère non-humain ou être administrée en mélange avec de la nourriture.

Selon un mode particulier, les compositions telles que décrites dans la présente demande comprennent en outre un excipient. Par « excipient » on entend tout ingrédient présent dans la composition qui n'est pas actif en tant que tel contre la cryptosporidiose, et qui est bien toléré par le mammifère non-humain, c'est-à-dire qui n'engendre pas d'effets secondaires. Comme exemples d'excipients, on peut citer, sans limitation, tous les excipients vétérinaires connus de l'homme du métier, en particulier ceux appartenant à la classe des solvants, des solubilisants, des tensioactifs, des antioxydants, des épaississants, des conservateurs, et des antimoussants.

Selon un mode préféré de l'invention, le au moins un excipient est choisi parmi la silice colloïdale anhydre, le glucose monohydraté, l'alcool benzylique, le métabisulfite de sodium, et l'édéate disodique.

Les compositions vétérinaires utilisées dans la présente invention peuvent être formulées sous n'importe quelle forme connue de l'homme du métier. Par exemple, les compositions peuvent être sous forme liquide, de préférence sous la forme d'une solution ou d'une émulsion. Les compositions peuvent aussi se présenter sous forme solide, de préférence sous forme d'un gel, d'une pâte, d'une poudre, d'un comprimé, d'une capsule, de granules, de gélules, ou de pilules. Bien entendu, l'homme du métier saura ajuster la proportion et la nature des excipients en fonction de la formulation et de la voie d'administration envisagées. Selon un mode particulier de l'invention, la composition est une poudre ou une solution. Selon un mode préféré de l'invention, la composition est une solution.

L'invention est adaptée au traitement de tout animal, et plus précisément de tout mammifère non-humain. Selon un mode particulier de l'invention, le mammifère non-humain est un bovin, un porcin, un caprin, ou un ovin, de préférence un bovin, avantageusement un veau. Selon un mode encore plus particulier de l'invention, le mammifère non-humain est un nouveau-né ou ayant un âge allant jusqu'à 21 jours, de préférence jusqu'à 14 jours. Selon un mode préféré, le mammifère non-humain a un âge de 2 ou 3 jours. Selon un autre mode préféré, le mammifère non-humain a un âge compris entre 6 et 13 jours, de préférence entre 7 et 10 jours. Selon un mode encore plus préféré de l'invention, le mammifère non-humain est un bovin ou un veau, nouveau-né ou ayant un âge allant jusqu' à 4 jours, de préférence de 2 ou 3 jours. Selon un autre mode encore plus préféré de l'invention, le mammifère non-humain est un bovin ou un veau ayant un âge allant jusqu'à 14 jours, de préférence compris entre 6 et 13 jours, et de manière encore plus préférée compris entre 7 et 10 jours.

Un objet préféré de l'invention est une composition vétérinaire comprenant du sulfate de paromomycine ou un de ses sels pharmaceutiquement acceptables destinée à être utilisée dans la prévention et/ou le traitement de la cryptosporidiose chez un bovin, dans laquelle la composition est administrée oralement audit bovin à une dose de sulfate de paromomycine comprise entre 114,3 et 200 mg/kg/jour, de préférence entre 120 et 200 mg/kg/jour, entre 120 et 180 mg/kg/jour, entre 130 et 170 mg/kg/jour, entre 140 et 160 mg/kg/jour, et d'une manière encore plus préférée d'environ 150 mg/kg/jour, pendant 3 à 6 jours, de préférence pendant 3 à 5 jours, et d'une manière encore plus préférée pendant 5 jours.

Un objet supplémentaire de l'invention concerne une composition vétérinaire pour son utilisation telle que décrite dans la présente demande, pour réduire et/ou supprimer l'excrétion des oocystes. L'invention concerne aussi une composition vétérinaire pour son utilisation telle que décrite dans la présente demande, pour réduire et/ou supprimer l'état de déshydratation. L'invention concerne également une composition vétérinaire pour son utilisation telle que décrite dans la présente demande, pour traiter et/ou prévenir la diarrhée. L'invention concerne encore une composition vétérinaire pour son utilisation telle que décrite dans la présente demande, pour augmenter le gain en poids journalier.

Un autre objet supplémentaire concerne une méthode pour réduire et/ou supprimer l'excrétion des oocystes comprenant l'administration d'une composition vétérinaire comprenant de la paromomycine ou un de ses sels pharmaceutiquement acceptables à une dose de paromomycine comprise entre 80 et 140 mg/kg/jour pendant 3 à 6 jours chez un mammifère non-humain souffrant de cryptosporidiose. Un autre objet concerne aussi une méthode pour réduire et/ou supprimer l'état de déshydratation comprenant l'administration d'une composition vétérinaire comprenant de la paromomycine ou un de ses sels pharmaceutiquement acceptables à une dose de paromomycine comprise entre 80 et 140 mg/kg/jour pendant 3 à 6 jours chez un mammifère non-humain souffrant de cryptosporidiose. Un autre objet concerne également une méthode pour traiter et/ou prévenir la diarrhée comprenant l'administration d'une composition vétérinaire comprenant de la paromomycine ou un de ses sels pharmaceutiquement acceptables à une dose de paromomycine comprise entre 80 et 140 mg/kg/jour pendant 3 à 6 jours chez un mammifère non-humain souffrant de cryptosporidiose. Un autre objet concerne encore une méthode pour augmenter le gain en poids journalier d'un mammifère non-humain souffrant de cryptosporidiose, comprenant l'administration d'une composition vétérinaire comprenant de la paromomycine ou un de ses sels pharmaceutiquement acceptables à une dose de paromomycine comprise entre 80 et 140 mg/kg/jour pendant 3 à 6 jours chez ledit mammifère non-humain.

D'autres aspects et avantages de l'invention apparaîtront à la lecture des exemples qui suivent, et qui doivent être considérés comme illustratifs et non limitatifs.

### EXEMPLES

### Matériel et méthodes

### Composition

La composition utilisée dans cette étude était une solution orale contenant 200 mg/mL de sulfate de paromomycine (correspondant à 140 mg/mL de paromomycine). Les différentes doses de 75, 100, et 150 mg/kg de sulfate de paromomycine ont été obtenues à partir de cette solution orale à 200 mg/mL de sulfate de paromomycine.

### Traitement

35 veaux âgés de 2 à 4 jours ont été inoculés par environ 10⁶ d'oocystes sporulés de *Cryptosporidium parvum.* Les veaux ont ensuite été répartis dans les 4 groupes suivants et traités dans les conditions suivantes à Dt0 (Jour 0) :
- 9 veaux non traités (groupe contrôle) ;
- 9 veaux traités avec 75 mg/kg/jour de sulfate de paromomycine pendant 5 jours (groupe 75) ;
- 9 veaux traités avec 100 mg/kg/jour de sulfate de paromomycine pendant 5 jours (groupe 100) ; et
- 9 veaux traités avec 150 mg/kg/jour de sulfate de paromomycine pendant 5 jours (groupe 150).

### Résultats

### 1. Signes cliniques

### Diarrhée (score fécal)

La consistance des fèces a été analysée à Dt0, puis deux fois par jours jusqu'à Dt5 (avant le repas du matin et dans l'après-midi). Un score de 0 à 2 a été attribué selon les observations suivantes :
- Score 0 : normal fèces
- Score 1 : diarrhée
- Score 2 : diarrhée aqueuse (sans fèces)

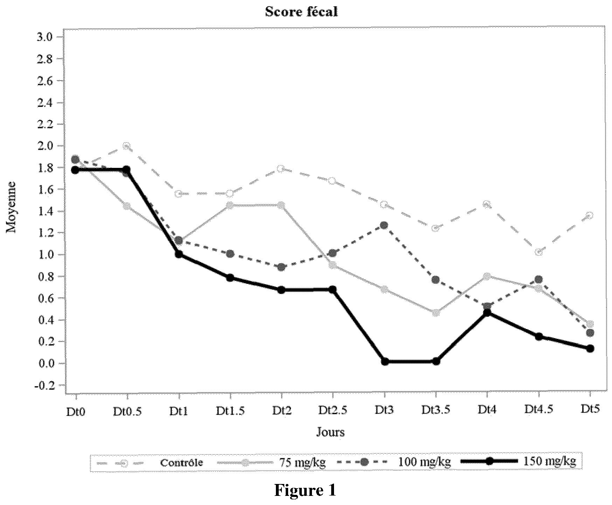

Les résultats de la figure 1 montrent un score fécal inférieur pour le groupe 150 par rapport aux groupes contrôle, 75 et 100, et ce dès le premier jour après le démarrage du traitement.

Les résultats du tableau 1 ci-dessous décrivent le score fécal pour chaque groupe au cours du traitement. Un meilleur score fécal (c'est-à-dire plus faible) est obtenu pour le groupe 150 après 5 jours de traitement.

**Tableau 1 :**

| **Score fécal** | | **Groupes** | | | |
|---|---|---|---|---|---|
| **Jour** | **Statistiques** | **Contrôle *(N=9)*** | **75 *(N=9)*** | **100 *(N=8)*** | **150 *(N=9)*** |
| *Dt0,0* | ***Moy. (+*/*-SD)*** | 1,78 (+/-0,44) | 1,89 (+/-0,33) | 1,88 (+/-0,35) | 1,78 (+/-0,44) |
| | ***Min ; Max*** | 1,00 ; 2,00 | 1,00 ; 2,00 | 1,00 ; 2,00 | 1,00 ; 2,00 |
| *Dt0,5* | ***Moy. (+*/*-SD)*** | 2,00 (+/-0,00) | 1,44 (+/-0,73) | 1,75 (+/-0,46) | 1,78 (+/-0,44) |
| | ***Min ; Max*** | 2,00 ; 2,00 | 0,00 ; 2,00 | 1,00 ; 2,00 | 1,00 ; 2,00 |
| *Dt1* | ***Moy. (+*/*-SD)*** | 1,56 (+/-0,73) | 1,11 (+/-0,60) | 1,13 (+/-0,83) | 1,00 (+/-0,71) |
| | ***Min ; Max*** | 0,00 ; 2,00 | 0,00 ; 2,00 | 0,00 ; 2,00 | 0,00 ; 2,00 |
| *Dt1,5* | ***Moy. (+*/*-SD)*** | 1,56 (+/-0.53) | 1,44 (+/-0.88) | 1,00 (+/-0,76) | 0,78 (+/-0,97) |
| | ***Min ; Max*** | 1,00 ; 2,00 | 0,00 ; 2,00 | 0,00 ; 2,00 | 0,00 ; 2,00 |
| *Dt2* | ***Moy. (+*/*-SD)*** | 1,78 (+/-0,44) | 1,44 (+/-0,73) | 0,88 (+/-0,83) | 0,67 (+/-0,87) |
| | ***Min ; Max*** | 1,00 ; 2,00 | 0,00 ; 2,00 | 0,00 ; 2,00 | 0,00 ; 2,00 |
| *Dt2,5* | ***Moy. (+*/*-SD)*** | 1,67 (+/-0,50) | 0,89 (+/-0,78) | 1,00 (+/-0,93) | 0,67 (+/-0,87) |
| | ***Min ; Max*** | 1,00 ; 2,00 | 0,00 ; 2,00 | 0,00 ; 2,00 | 0,00 ; 2,00 |
| *Dt3* | ***Moy. (+*/*-SD)*** | 1,44 (+/-0.53) | 0,67 (+/-0.71) | 1,25 (+/-0.71) | 0,00 (+/-0.00) |
| | ***Min ; Max*** | 1,00 ; 2,00 | 0,00 ; 2,00 | 0,00 ; 2,00 | 0,00 ; 0,00 |
| *Dt3,5* | ***Moy. (+*/*-SD)*** | 1,22 (+/-0,44) | 0,44 (+/-0,53) | 0,75 (+/-0,46) | 0,00 (+/-0,00) |
| | ***Min ; Max*** | 1,00 ; 2,00 | 0,00 ; 1,00 | 0,00 ; 1,00 | 0,00 ; 0,00 |
| *Dt4* | ***Moy. (+*/*-SD)*** | 1,44 (+/-0,73) | 0,78 (+/-0,67) | 0,50 (+/-0,53) | 0,44 (+/-0,53) |
| | ***Min ; Max*** | 0,00 ; 2,00 | 0,00 ; 2,00 | 0,00 ; 1,00 | 0,00 ; 1,00 |
| *Dt4,5* | ***Moy. (+*/*-SD)*** | 1,00 (+/-0,71) | 0,67 (+/-0,50) | 0,75 (+/-0,89) | 0,22 (+/-0,44) |
| | ***Min ; Max*** | 0,00 ; 2,00 | 0,00 ; 1,00 | 0,00 ; 2,00 | 0,00 ; 1,00 |
| *Dt5* | ***Moy. (+*/*-SD)*** | 1,33 (+/-0,50) | 0,33 (+/-0,50) | 0,25 (+/-0,46) | 0,11 (+/-0,33) |
| | ***Min ; Max*** | 1,00 ; 2,00 | 0,00 ; 1,00 | 0,00 ; 1,00 | 0,00 ; 1,00 |

| | | | | | |
|---|---|---|---|---|---|
| Moy. : Moyenne | | | | | |

### Score sur l'état général sur la santé

L'état général sur la santé des veaux a été observé à Dt0 puis deux fois par jour jusqu'à Dt5 (avant le repas du matin et dans l'après-midi). Un score de 0 à 3 a été attribué selon les observations suivantes :
- Score 0 : veau alerte et actif
- Score 1 : veau moyennement dépressif (oreilles tombantes et légèrement insensible aux stimuli
- Score 2 : veau dépressif (oreilles et tête tombantes et pas d'intérêt à se tenir debout)
- Score 3 : veau couché (incapable de se tenir debout)

Les résultats du tableau 2 ci-dessous décrivent l'évolution de l'état général sur la santé des veaux pour chaque groupe au cours du traitement. Un meilleur score de l'état général sur la santé est obtenu pour le groupe 150, et ce dès les premières 12 heures après le démarrage du traitement.

**Tableau 2 :**

| **Etat général sur la santé** | | **Groupes** | | | |
|---|---|---|---|---|---|
| **Jour** | **Statistiques** | **Contrôle *(N=9)*** | **75 *(N=9)*** | **100 *(N=8)*** | **150 *(N=9)*** |
| *Dt0* | ***Moy. (+*/*-SD)*** | 0,44 (+/-0,53) | 0,56 (+/-0,73) | 0,75 (+/-0,46) | 0,00 (+/-0,00) |
| | ***Min ; Max*** | 0,00 ; 1.00 | 0,00 ; 2,00 | 0,00 ; 1,00 | 0,00 ; 0,00 |
| *Dt0,5* | ***Moy. (+*/*-SD)*** | 0,44 (+/-0,53) | 0,89 (+/-1,05) | 0,50 (+/-0,53) | 0,00 (+/-0,00) |
| | ***Min ; Max*** | 0,00 ; 1,00 | 0,00 ; 3,00 | 0,00 ; 1,00 | 0,00 ; 0,00 |
| *Dt1* | ***Moy. (+*/*-SD)*** | 0,33 (+/-0,50) | 0,44 (+/-0,53) | 0,38 (+/-0,52) | 0,22 (+/-0,44) |
| | ***Min ; Max*** | 0,00 ; 1,00 | 0,00 ; 1,00 | 0,00 ; 1,00 | 0,00 ; 1,00 |
| *Dt1,5* | ***Moy. (+*/*-SD)*** | 0,56 (+/-0,53) | 0,22 (+/-0,44) | 0,25 (+/-0,46) | 0,33 (+/-0,50) |
| | ***Min ; Max*** | 0,00 ; 1,00 | 0,00 ; 1,00 | 0,00 ; 1,00 | 0,00 ; 1,00 |
| *Dt2* | ***Moy. (+*/*-SD)*** | 0,78 (+/-0,44) | 0,22 (+/-0,44) | 0,50 (+/-0,53) | 0,00 (+/-0,00) |
| | ***Min ; Max*** | 0,00 ; 1,00 | 0,00 ; 1,00 | 0,00 ; 1,00 | 0,00 ; 0,00 |
| *Dt2,5* | ***Moy. (+*/*-SD)*** | 0,56 (+/-0,53) | 0,22 (+/-0,44) | 0,50 (+/-0,53) | 0,00 (+/-0,00) |
| | ***Min ; Max*** | 0,00 ; 1,00 | 0,00 ; 1,00 | 0,00 ; 1,00 | 0,00 ; 0,00 |
| *Dt3* | ***Moy. (+*/*-SD)*** | 0,67 (+/-0,50) | 0,11 (+/-0,33) | 0,00 (+/-0,00) | 0,00 (+/-0,00) |
| | ***Min ; Max*** | 0,00 ; 1,00 | 0,00 ; 1,00 | 0,00 ; 0,00 | 0,00 ; 0,00 |
| *Dt3,5* | ***Moy. (+*/*-SD)*** | 0,56 (+/-0,73) | 0,00 (+/-0,00) | 0,13 (+/-0,35) | 0,00 (+/-0,00) |
| | ***Min ; Max*** | 0,00 ; 2.00 | 0,00 ; 0.00 | 0,00 ; 1,00 | 0,00 ; 0,00 |
| *Dt4* | ***Moy. (+*/*-SD)*** | 0,67 (+/-0.71) | 0,11 (+/-0.33) | 0,00 (+/-0.00) | 0,00 (+/-0.00) |
| | ***Min ; Max*** | 0,00 ; 2,00 | 0,00 ; 1,00 | 0,00 ; 0,00 | 0,00 ; 0,00 |
| *Dt4,5* | ***Moy. (+*/*-SD)*** | 0,56 (+/-0,53) | 0,00 (+/-0,00) | 0,00 (+/-0,00) | 0,00 (+/-0,00) |
| | ***Min ; Max*** | 0,00 ; 1,00 | 0,00 ; 0,00 | 0,00 ; 0,00 | 0,00 ; 0,00 |
| *Dt5* | ***Moy. (+*/*-SD)*** | 0,44 (+/-0,53) | 0,22 (+/-0,44) | 0,00 (+/-0,00) | 0,00 (+/-0,00) |
| | ***Min ; Max*** | 0,00 ; 1,00 | 0,00 ; 1,00 | 0,00 ; 0,00 | 0,00 ; 0,00 |

| | | | | | |
|---|---|---|---|---|---|
| Moy. : Moyenne | | | | | |

### Score d'hydratation

Le score d'hydratation a été mesuré une fois par jour avant le repas du matin à partir de Dt0 jusqu'à Dt5. Un score de 0 à 3 a été attribué selon les observations suivantes :
- Score 0 : statut normal d'hydratation, plis de peau < 1 seconde
- Score 1 : légère déshydratation, yeux légèrement déprimés, et plis de peau ≤ 2 secondes
- Score 2 : déshydratation modérée, yeux déprimés, museau sec, plis de peau > 2 secondes et < 5 secondes
- Score 3 : déshydratation sévère, sévère énophtalmie, plis de peau > 5 secondes

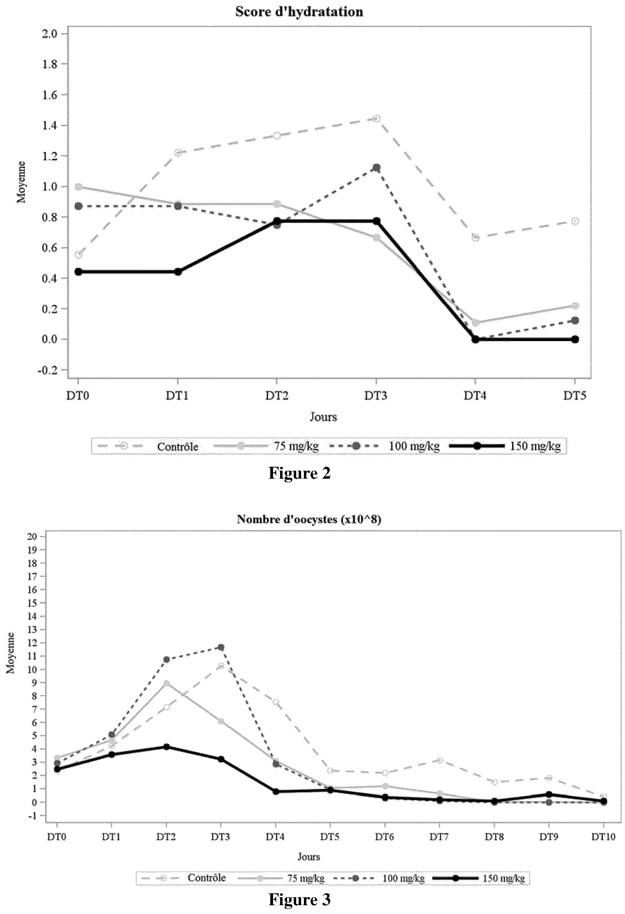

Les résultats de la figure 2 montrent un score d'hydratation inférieur (c'est-à-dire une meilleure hydratation) pour le groupe 150 par rapport aux groupes contrôle, 75 et 100.

### Guérison clinique

La guérison clinique a été calculée à partir des données cliniques présentées ci-dessus pour permettre de statuer sur l'efficacité des doses testées. Un veau est considéré comme étant guéri lorsqu'il a un score fécal égal à 0, un score sur l'état général sur la santé égal à 0 et un score d'hydratation égal à 0.

Les résultats du tableau 3 ci-dessous décrivent l'évolution de la guérison clinique des veaux pour chaque groupe au cours du traitement. Une meilleure guérison clinique est observée pour le groupe 150 au cours du traitement.

**Tableau 3 :**

| **Guérison clinique** | | **Groupes** | | | |
|---|---|---|---|---|---|
| **Jour** | **Statistiques** | **Contrôle *(N=9)*** | **75 *(N=9)*** | **100 *(N=8)*** | **150 *(N=9)*** |
| *Dt0* | ***n(%)*** | 0 (0,0%) | 0 (0,0%) | 0 (0,0%) | 0 (0,0%) |
| *Dt0,5* | ***n(%)*** | 0 (0,0%) | 1 (11,1%) | 0 (0,0%) | 0 (0,0%) |
| *Dt1* | ***n(%)*** | 0 (0,0%) | 1 (11,1%) | 1 (12,5%) | 1 (11,1%) |
| *Dt1,5* | ***n(%)*** | 0 (0,0%) | 1 (11,1%) | 0 (0,0%) | 1 (11,1%) |
| *Dt2* | ***n(%)*** | 0 (0,0%) | 0 (0,0%) | 0 (0,0%) | 2 (22,2%) |
| *Dt2,5* | ***n(%)*** | 0 (0,0%) | 1 (11,1%) | 0 (0,0%) | 2 (22,2%) |
| *Dt3* | ***n(%)*** | 0 (0,0%) | 2 (22,2%) | 0 (0,0%) | 2 (22,2%) |
| *Dt3,5* | ***n(%)*** | 0 (0,0%) | 5 (55,6%) | 1 (12,5%) | 9 *(100,0%)* |
| *Dt4* | ***n(%)*** | 1 (11,1%) | 3 (33,3%) | 4 (50,0%) | 5 (55,6%) |
| *Dt4,5* | ***n(%)*** | 2 (22,2%) | 3 (33,3%) | 3 (37,5%) | 7 (77,8%) |
| *Dt5* | ***n(%)*** | **0 (0,0%)** | **4 (44,4%)** | **5 (62,5%)** | **8 (88,9%)** |

### 2. Nombre d'oocystes

Des échantillons de fèces ont été prélevés tous les jours le matin à partir de Dt0 jusqu'à Dt10. L'analyse quantitative des oocystes de *Cryptosporidium parvum* a été réalisée avec le test utilisant le réactif Merifluor^{®} de chez Meridian Diagnostics.

Les résultats de la figure 3 montrent un nombre d'oocystes présents dans les échantillons très inférieur pour le groupe 150 par rapport aux groupes contrôle, 75 et 100. Ceci démontre que le groupe 150 excrète donc moins d'oocystes que les groupes contrôle, 75 et 100.

### 3. Poids corporel

La moyenne du gain de poids journalier des veaux a également été calculée de Dt0 jusqu'à Dt21 pour chaque groupe. La moyenne de ce gain de poids journalier est représentée dans le tableau 4 ci-dessous. Un meilleur gain en poids journalier est observé pour le groupe 150 au cours du traitement.

**Tableau 4 :**

| **Paramètre** | **Statistiques** | **Groupes** | | | |
|---|---|---|---|---|---|
| | | **Contrôle *(N=9)*** | **75 *(N=9)*** | **100 *(N=8)*** | **150 *(N=9)*** |
| **Gain en poids journalier (kg)** | ***Moy. (+*/*-SD)*** | 0,53 (+/-0.16) | 0,56 (+/-0.19) | 0,56 (+/-0.13) | 0,67 (+/-0.06) |
| | ***Min ; Max*** | 0,18 ; 0,77 | 0,15 ; 0.81 | 0,35 ; 0.69 | 0,58 ; 0,77 |

### Conclusions

Les exemples présentés ci-dessus montrent qu'un traitement comprenant l'administration d'une dose de sulfate de paromomycine à 150 mg/kg/jour (ou 105 mg/kg/jour de paromomycine) permet d'améliorer les signes cliniques (diarrhée, état général sur la santé, hydratation), de réduire l'excrétion des oocystes, et d'augmenter le gain en poids journalier chez un veau inoculé par des oocystes de *Cryptosporidium parvum.* Les inventeurs ont donc démontré de manière surprenante que l'utilisation de la paromomycine ou un de ses sels pharmaceutiquement acceptables à une dose en paromomycine comprise entre 80 et 140 mg/kg/jour pendant 3 à 6 jours permettait de traiter intégralement et de manière plus efficace et plus rapide la cryptosporidiose ainsi que les signes cliniques associées à cette maladie

## Revendications

1. Composition vétérinaire comprenant de la paromomycine ou un de ses sels pharmaceutiquement acceptables destinée à être utilisée dans la prévention et/ou le traitement de la cryptosporidiose chez un mammifère non-humain, dans laquelle la composition est administrée audit mammifère non-humain à une dose de paromomycine comprise entre 80 et 140 mg/kg/jour pendant 3 à 6 jours.

2. Composition vétérinaire destinée à être utilisée selon la revendication 1, dans laquelle la dose de paromomycine est comprise entre 84 et 126 mg/kg/jour, entre 91 et 119 mg/kg/jour, de préférence entre 98 et 112 mg/kg/jour, et de manière encore plus préférée est d'environ 105 mg/kg/jour.

3. Composition vétérinaire destinée à être utilisée selon la revendication 1 ou 2, dans laquelle le sel pharmaceutiquement acceptable de la paromomycine est un sulfate.

4. Composition vétérinaire comprenant du sulfate de paromomycine destinée à être utilisée dans la prévention et/ou le traitement de la cryptosporidiose chez un mammifère non-humain, dans laquelle la composition est administrée audit mammifère non-humain à une dose de sulfate de paromomycine comprise entre 114,3 et 200 mg/kg/jour, de préférence d'environ 150 mg/kg/jour, pendant 3 à 6 jours.

5. Composition vétérinaire destinée à être utilisée selon l'une quelconque des revendications 1 à 4, dans laquelle la composition est administrée pendant 3 à 5 jours, de préférence pendant 5 jours.

6. Composition vétérinaire destinée à être utilisée selon l'une quelconque des revendications 1 à 5, dans laquelle la composition est administrée en une seule prise par jour.

7. Composition vétérinaire destinée à être utilisée selon l'une quelconque des revendications 1 à 6, dans laquelle la composition est administrée par voie orale.

8. Composition vétérinaire destinée à être utilisée selon l'une quelconque des revendications 1 à 7, dans laquelle le mammifère non-humain est un bovin, un porcin, un caprin, ou un ovin, de préférence un bovin, et de manière encore plus préférée un veau.

9. Composition vétérinaire destinée à être utilisée selon l'une quelconque des revendications 1 à 8, dans laquelle le mammifère non-humain est un nouveau-né ou ayant un âge allant jusqu'à 21 jours, de préférence jusqu'à 14 jours, de manière encore plus préférée ayant un âge de 2 ou 3 jours ou un âge compris entre 6 et 13 jours, de préférence entre 7 et 10 jours.

10. Composition vétérinaire destinée à être utilisée selon l'une quelconque des revendications 1 à 9, dans laquelle la composition comprend en outre au moins un excipient.

11. Composition vétérinaire destinée à être utilisée selon l'une quelconque des revendications 1 à 10, dans laquelle la composition est une solution.

12. Composition vétérinaire destinée à être utilisée selon l'une quelconque des revendications 1 à 11, pour réduire et/ou supprimer l'excrétion des oocystes.

13. Composition vétérinaire destinée à être utilisée selon l'une quelconque des revendications 1 à 11, pour réduire et/ou supprimer l'état de déshydratation.

14. Composition vétérinaire destinée à être utilisée selon l'une quelconque des revendications 1 à 11, pour traiter et/ou prévenir la diarrhée.

15. Composition vétérinaire destinée à être utilisée selon l'une quelconque des revendications 1 à 11, pour augmenter le gain en poids journalier.
